(19) **Europäisches Patentamt European Patent Office Office européen des brevets**

(11) **EP 2 407 848 B1**

(12) **FASCICULE DE BREVET EUROPEEN**

(45) Date de publication et mention
de la délivrance du brevet:
**13.02.2013 Bulletin 2013/07**

(51) Int Cl.:
*G05D 21/02* (2006.01)   *C12M 1/40* (2006.01)
*F03G 7/00* (2006.01)   *G05D 21/00* (2006.01)

(21) Numéro de dépôt: **11183185.5**

(22) Date de dépôt: **03.12.2007**

(54) **Applications d'un dispositif de variation du pH d'une solution**

Anwendungen einer Vorrichtung zum Ändern des pH-Wertes einer Lösung

Uses of a device for varying the pH of a solution

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **04.12.2006 FR 0655296**

(43) Date de publication de la demande:
**18.01.2012 Bulletin 2012/03**

(62) Numéro(s) de document de la (des) demande(s)
initiale(s) en application de l'article 76 CBE:
**07870383.2 / 2 100 202**

(73) Titulaires:
• **Université Joseph Fourier**
  **38041 Grenoble Cedex 9 (FR)**
• **Institut National des Sciences Appliquées de Toulouse**
  **31077 Toulouse Cedex 4 (FR)**

(72) Inventeurs:
• **Cinquin, Philippe**
  **38330 Saint Nazaire les Eymes (FR)**
• **Lenouvel, François**
  **38100 Grenoble (FR)**
• **Durrieu, Vanessa**
  **31400 Toulouse (FR)**
• **Mathe, Stéphane**
  **31500 Toulouse (FR)**
• **Mozer, Pierre**
  **94300 Vincennes (FR)**

(74) Mandataire: **de Beaumont, Michel**
  **1bis, rue Champollion**
  **38000 Grenoble (FR)**

(56) Documents cités:
**WO-A2-03/072941   DE-C1- 19 728 663
FR-A1- 2 881 481**

**Description**

Domaine de l'invention

La présente invention concerne des applications d'un dispositif de variation du pH d'une solution.

Exposé de l'art antérieur

Pour certaines applications, il est souhaitable de pouvoir modifier le pH d'une solution. Un exemple d'application concerne la régulation d'un phénomène physico-chimique se produisant en solution et dont la vitesse varie en fonction du pH de la solution, par exemple, la variation de solubilisation d'une substance dans une solution dont on fait varier le pH. Un autre exemple d'application concerne la régulation de propriétés chimiques ou mécaniques d'une solution qui varient en fonction du pH. Il s'agit, par exemple, de l'osmolarité de la solution.

La modification du pH d'une solution initiale peut être obtenue, de façon classique, en mélangeant à la solution initiale une solution supplémentaire acide ou basique. Le pH de la solution finalement obtenue après mélange se stabilise à une valeur qui dépend du pH de la solution initiale, du pH de la solution supplémentaire et des volumes des solutions initiale et supplémentaire.

Toutefois, pour certaines applications, il serait souhaitable de pouvoir modifier le pH d'une solution sans devoir y ajouter une solution acide ou basique. A titre d'exemple, il serait souhaitable de pouvoir modifier le pH d'une solution contenue dans le corps humain, appelée par la suite solution biologique, en limitant le plus possible toute intervention au niveau du corps humain pour réaliser la modification de pH.

Les documents WO 03/072941 et FR 2 881 481 décrivent un actionneur. Le document DE 197 28 663 décrit un biocapteur pour la détermination quantitative de formaldéhyde.

Résumé de l'invention

La présente invention vise des applications d'un dispositif de variation du pH d'une solution qui ne nécessitent pas l'ajout d'une solution acide ou basique et plus particulièrement d'un dispositif de variation de pH susceptible d'être implanté au niveau du corps humain.

Pour atteindre ces objets, la présente invention prévoit des applications d'un dispositif de variation du pH d'un solvant dans lequel est dissous une substance tel que définies dans les revendications ci-après, le dispositif de variation du pH étant par exemple du type décrit dans DE-197 28 663.

Brève description des dessins

Ces objets, caractéristiques et avantages, ainsi que d'autres de la présente invention seront exposés en détail dans la description suivante d'exemples de réalisation particuliers faite à titre non-limitatif en relation avec les figures jointes parmi lesquelles :

la figure 1 représente un premier exemple de réalisation d'un dispositif de variation de pH ;
la figure 2 représente un second exemple de réalisation du dispositif de variation de pH ;
les figures 3A et 3B représentent deux étapes de fonctionnement d'un exemple de réalisation d'un actionneur osmotique selon l'invention mettant en oeuvre le dispositif de variation de pH selon le premier exemple de réalisation ;
les figures 4A à 4D représentent quatre étapes de fonctionnement d'un premier exemple de réalisation d'un moteur osmotique selon l'invention, mettant en oeuvre le premier exemple de réalisation du dispositif de variation de pH;
les figures 5A et 5B représentent deux étapes de fonctionnement d'un second exemple de réalisation d'un moteur osmotique selon l'invention, mettant en oeuvre le second exemple de réalisation du dispositif de variation de pH;
les figures 6A et 6B représentent deux étapes de fonctionnement d'une variante du second exemple de réalisation du moteur osmotique ;
les figures 7A et 7B représentent deux étapes de fonctionnement d'un dispositif d'épuration selon l'invention mettant en oeuvre le premier exemple de réalisation du dispositif de variation de pH; et
les figures 8A à 8D représentent quatre étapes de fonctionnement d'un troisième exemple de réalisation d'un moteur osmotique selon l'invention, mettant en oeuvre le premier exemple de réalisation du dispositif de variation de pH.

Description détaillée

De mêmes éléments ont été désignés par les mêmes références aux différentes figures. Pour des raisons de clarté, seuls les éléments qui sont utiles à la compréhension de l'invention ont été représentés aux figures et seront décrits par la suite.

Il est prévu de favoriser une première série ou une seconde série de réactions chimiques dans une solution dont on souhaite modifier le pH, la première série de réactions chimiques entraînant une diminution du pH de la solution tandis que la seconde série de réactions chimiques entraîne une augmentation du pH de la solution.

Toute réaction aboutissant à la formation d'ions H$^+$, et donc à une diminution du pH, peut convenir pour la première série de réactions. C'est en particulier le cas de l'oxydation du D-glucose, ou stéréoisomère D du glucose, par l'enzyme glucose oxydase, qui aboutit à la formation d'acide gluconique (susceptible de libérer un ion H$^+$) et d'eau oxygénée. L'eau oxygénée est de plus dégradable par l'enzyme catalase ou l'enzyme peroxydase pour fournir des ions H$^+$ supplémentaires. Les réactions mises en oeuvre sont les suivantes:

$$D\text{-}glucose + H_2O + O_2 \xrightarrow{\text{glucose oxydase}} D\text{-}acide\ gluconique + H_2O_2$$

$$D\text{-}acide\ gluconique \longrightarrow D\text{-}gluconate + H^+$$

$$H_2O_2 \xrightarrow{\text{catalase/peroxidase}} 2H^+ + O_2 + 2e^- \qquad (1)$$

La première série de réactions peut correspondre à l'oxydation du L-glucose, ou stéréoisomère L du glucose, par l'enzyme L-Fucose déshydrogénase. Les réactions mises en oeuvre sont les suivantes:

$$L\text{-}glucose + NADP \xrightarrow[\text{deshydrogénase}]{\text{L-fucose}} L - glucono - 1,5 - lactone + NADPH$$

$$L - glucono - 1,5 - lactone + H_2O \longrightarrow L\text{-}acide\ gluconique + H^+ \qquad (2)$$

où le composé NADP correspond au Nicotinamide Adénine Dinucléotide Phosphate et où le composé NADPH correspond au même composé une fois réduit.

La seconde série de réactions mises en oeuvre pour rendre basique une solution peut correspondre à la dégradation de l'urée par l'enzyme uréase qui aboutit à la formation d'ions ammonium $NH_4^+$ et d'ions hydroxyle $OH^-$, c'est-à-dire à une augmentation du pH. Les réactions mises en oeuvre sont les suivantes:

$$NH_2 - CO - NH_2 + H_2O \xrightarrow{\text{uréase}} CO_2 + 2NH_3$$

$$2NH_3 + H_2O \longrightarrow 2NH_4^+ + 2OH^- \qquad (3)$$

Les réactions (1) et (3) présentent l'avantage de pouvoir être directement mises en oeuvre avec une solution biologique qui contient naturellement du D-glucose, qui est le glucose participant à la glycémie, et de l'urée. Les réactions (2) peuvent facilement être mises en oeuvre avec une solution biologique qui contient naturellement le composé NADP. Il suffit alors d'ajouter du L-glucose à la solution biologique.

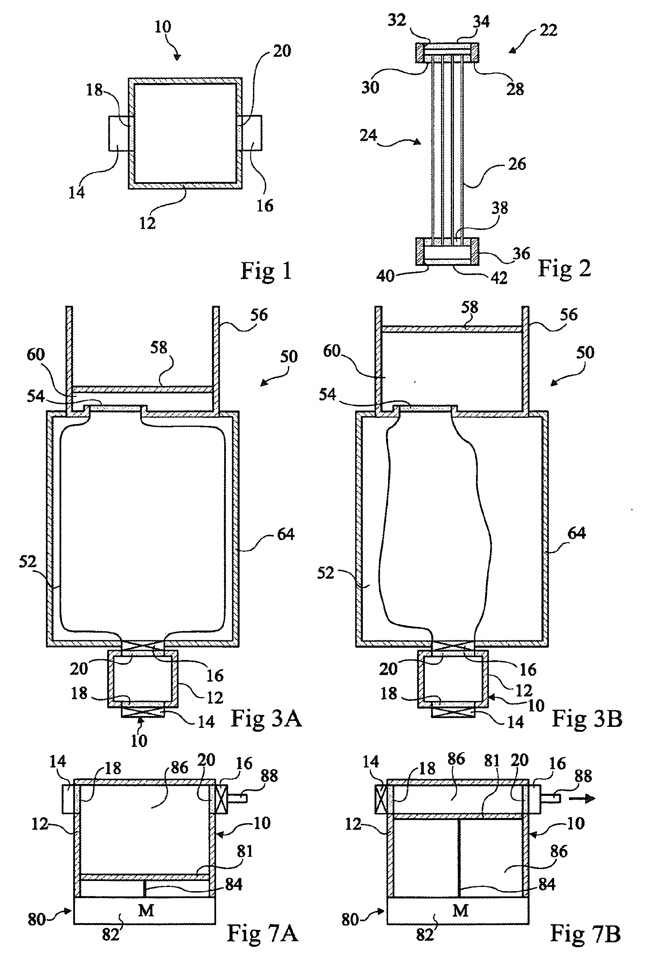

La figure 1 représente un premier exemple de réalisation d'un dispositif 10 de variation de pH. Le dispositif 10 est disposé dans une solution dont on souhaite modifier le pH, par exemple une solution biologique. Le dispositif 10 comprend une enceinte 12 étanche et une vanne 14 qui, lorsqu'elle est ouverte, met en communication le contenu de l'enceinte 12 avec la solution extérieure. Le dispositif 10 peut être relié à un système, non représenté, dont le fonctionnement nécessite l'apport d'une solution ayant un pH compris dans une plage déterminée. Pour ce faire, le dispositif 10 comprend une vanne 16 qui, lorsqu'elle est ouverte, met en communication le contenu de l'enceinte 12 avec le système à alimenter. Le dispositif 10 comprend une membrane 18 disposée entre la vanne 14 et l'enceinte 12 et une membrane 20 disposée entre la vanne 16 et l'enceinte 12. Les membranes 18 et 20 ont des seuils de coupure donnés, exprimés généralement en daltons, c'est-à-dire qu'elles laissent passer des particules ayant une masse moléculaire sensiblement inférieure au seuil de coupure.

Lorsqu'on souhaite acidifier la solution contenue dans l'enceinte 12, on peut favoriser dans l'enceinte 12 la réaction d'oxydation du glucose, par exemple selon les réactions (1). Pour ce faire, le dispositif 10 est placé dans une solution contenant du D-glucose, par exemple une solution biologique, et on dispose dans l'enceinte 12 des enzymes glucose oxydase et des enzymes catalase ou peroxydase. Les membranes 18 et 20 ont des seuils de coupure tels qu'elles permettent de retenir les enzymes glucose oxydase et les enzymes catalase ou peroxydase dans l'enceinte 12. En outre, la membrane 18 a un seuil de coupure suffisamment élevé pour laisser passer le glucose. A titre d'exemple, les membranes 18 et 20 ont un seuil de coupure de l'ordre de quelques centaines de Daltons.

Le fonctionnement du premier exemple de dispositif 10 va maintenant être décrit pour une opération de diminution du pH. Initialement, la vanne 16 est fermée et la vanne 14 est ouverte permettant la diffusion du D-glucose dans l'enceinte 12. La vanne 14 est alors fermée. Le D-glucose contenu dans l'enceinte 12 est alors oxydé pour fournir des ions $H^+$ et de l'acide gluconique selon les réactions (1) précédemment décrites. Le pH de la solution contenue dans l'enceinte 12 diminue donc. La solution acide obtenue peut alors être utilisée par le système relié à l'enceinte 12 en ouvrant la vanne 16. La demanderesse a mis en évidence que pour une solution aqueuse ayant une concentration en D-glucose initiale de 5,5 mmol/l, ce qui correspond à la concentration moyenne de glucose dans le corps humain, une concentration d'enzyme glucose oxydase de 1 mg.ml$^{-1}$ soit 47 unités et un pH initial de 7, on obtient dans l'enceinte 12 un pH égal à 5 en l'espace d'une heure et un pH de 3,5 après trois heures.

L'acide gluconique issu de l'oxydation du glucose tend à former des sels, ou gluconate, avec les substances présentes en solution. S'il n'est pas souhaitable que le gluconate pénètre au niveau du système relié au dispositif 10, on choisit la membrane 20 avec un seuil de coupure suffisamment bas pour retenir le gluconate dans l'enceinte 12 lorsque la vanne 16 est ouverte. Le seuil de coupure de la membrane 20 est alors de l'ordre de 100 Daltons. A la prochaine ouverture de la vanne 14, le gluconate diffuse hors de l'enceinte 12. Dans le cas d'une application médicale pour laquelle le dispositif 10 est placé dans le corps humain, il est souhaitable que les enzymes catalase ou peroxydase soient disposées au niveau de la membrane 18 pour éviter le rejet dans le corps humain de radicaux libres issus de l'oxydation du D-glucose. Par ailleurs, le rejet de gluconate dans le corps humain ne présente pas de danger puisqu'il est naturellement évacué par les reins.

Pour une opération d'acidification d'une solution, on peut disposer dans l'enceinte 12 des enzymes L-fucose déshydrogénase, retenues par les membranes 18 et 20. Le dispositif 10 est alors placé dans une solution contenant du L-glucose, le fonctionnement du dispositif étant identique à ce qui a été décrit précédemment. Une solution biologique humaine ne comporte pas naturellement de L-glucose. Dans le cas où le dispositif 10 est placé dans le corps humain, on peut prévoir une étape d'ajout de L-glucose à la solution biologique, par exemple par injection par voie intraveineuse.

Le dispositif 10 selon le premier exemple de réalisation peut également être mis en oeuvre pour obtenir une augmentation de pH. Le dispositif 10 est alors disposé dans une solution contenant de l'urée, par exemple une solution biologique, et on favorise, dans l'enceinte 12, la dégradation de l'urée en ammoniaque et en acide carbonique selon les réactions (3). L'ammoniaque réagissant avec l'eau pour fournir des ions ammonium, on obtient ainsi une augmentation du pH. Pour ce faire, on dispose des enzymes uréase dans l'enceinte 12. Dans ce cas, les membranes 18 et 16 ont un seuil de coupure suffisamment bas pour retenir les enzymes uréase dans l'enceinte 12. En outre, la membrane 18 a un seuil de coupure suffisamment élevé pour laisser passer l'urée qui a une masse molaire de 60 g/mol. A titre d'exemple, la membrane 18 a un seuil de coupure de quelques centaines de Daltons. Le cycle de fonctionnement du dispositif 10 est alors identique à ce qui a été précédemment décrit. La demanderesse a mis en évidence que pour une solution ayant une concentration en urée de 3 mmol/l, ce qui correspond à la concentration moyenne d'urée dans le corps humain, et un pH initial de 7, on obtient dans l'enceinte 12 un pH égal à 8,2 en l'espace de deux heures et un pH de 9,2 après une nuit. Dans le cas d'une application médicale pour laquelle le dispositif 10 est placé dans le corps humain, le dioxyde de carbone issu des réactions (3) est naturellement évacué par la respiration. En outre, les ions ammonium issus des réactions (3) sont métabolisés par le foie.

La figure 2 représente un second exemple de réalisation d'un dispositif 22 de variation de pH. Le dispositif 22 comprend une enceinte 24 constituée d'un faisceau de fibres creuses à paroi semi-perméable 26, par exemple du type utilisé pour les opérations de dialyse. Chaque fibre a, par exemple, un diamètre de l'ordre de 200 $\mu$m. Le faisceau de fibres 26 est maintenu à une première extrémité par une première bague de jonction 28, par exemple par l'intermédiaire d'une zone de collage 30. La bague 28 comprend une ouverture 32 fermée par un bouchon 34. La seconde extrémité du faisceau de fibre 26 est maintenue par une seconde bague de jonction 36, par exemple par l'intermédiaire d'une zone de collage 38. La seconde bague 36 comprend une ouverture 40 fermée par un bouchon 42.

Le dispositif 22 est destiné à être plongé dans la solution dont on souhaite modifier le pH. Le diamètre et la longueur des fibres sont adaptés selon la surface d'échange désirée entre le faisceau de fibres 26 et la solution dans laquelle le dispositif 22 est plongé. Si l'on souhaite diminuer le pH, on dispose dans les fibres 26 des enzymes glucose oxydase et des enzymes catalase ou peroxydase par l'intermédiaire des ouvertures 32, 40 qui sont ensuite refermées par les bouchons 34, 42. La paroi des fibres 26 a alors un seuil de coupure tel qu'elle ne laisse pas passer les enzymes glucose oxydase et les enzymes catalase ou peroxydase qui sont retenues dans le faisceau de fibres 26 tandis qu'elle permet le passage du D-glucose et du gluconate. Si l'on souhaite augmenter le pH, on dispose dans les fibres 26 des enzymes uréase par l'intermédiaire des ouvertures 32, 40 qui sont ensuite refermées par les bouchons 34, 42. La paroi des fibres 26 a alors un seuil de coupure tel qu'elle ne laisse pas passer les enzymes uréase qui sont retenues dans le faisceau de fibres 26 tandis qu'elle permet le passage de l'urée.

Lorsque le dispositif 22 est implanté dans le corps humain pour acidifier une solution biologique, les enzymes catalase ou peroxydase sont de préférence fixées au niveau de la paroi interne des fibres 26 tandis que l'enzyme glucose oxydase peut être laissée libre dans le faisceau de fibres 26. Un tel agencement permet d'éviter que les radicaux libres créés par la dégradation du glucose par les enzymes D-glucose oxydase traversent la paroi des fibres 26 et se répandent dans la solution biologique.

Lorsque le dispositif 10, ou le dispositif 22, est utilisé pour acidifier une solution en mettant en oeuvre l'oxydation du D-glucose, il permet parallèlement la suppression d'une partie du D-glucose contenu dans la solution. En effet, les réactions mises en oeuvre dans l'enceinte 12 ou dans le faisceau de fibres 26 (réactions (1)) conduisent à la transformation du glucose en acide gluconique qui donne du gluconate. Un tel dispositif 10 (ou 22) peut être implanté dans le corps humain afin de supprimer un excès de D-glucose, généralement associé à un diabète de type I et de type II ou à une surcharge pondérale. Plusieurs lieux d'implantation du dispositif 10 (ou 22) sont alors envisageables. A titre d'exemple, le dispositif 10 (ou 22) peut être placé au niveau du péritoine ou d'un espace péri-cellulaire graisseux. Une implantation à proximité du péritoine présente l'avantage d'une riche vascularisation et d'une concentration élevée en D-glucose. Une implantation dans un espace graisseux diminue les risques de réactions inflammatoires. Le dispositif 10 (ou 22) peut également être implanté au niveau d'un muscle, par une ponction réalisée au moyen d'un trocart médical.

Dès son implantation dans le corps humain, le dispositif 22 transforme quotidiennement une certaine quantité de D-glucose en gluconate qui est naturellement éliminé par le rein. Le dispositif 10 permet, quant à lui, par la commande des vannes 14, 16, de contrôler la quantité de D-glucose transformée en gluconate. Par l'intermédiaire des vannes 14, 16 (respectivement des ouvertures 32, 40), il est possible d'accéder au contenu de l'enceinte 12 (respectivement des fibres 26) de façon à renouveler la population enzymatique contenue dans l'enceinte 12 (respectivement dans les fibres 26) si cela est nécessaire. Les chambres implantables utilisées classiquement pour les chimiothérapies permettent une ponction percutanée très facile, grâce à laquelle ce renouvellement peut être réalisé. Ceci permet de pallier une baisse d'activité enzymatique. Par ailleurs, l'accès au contenu de l'enceinte 12 (respectivement des fibres 26) permet également de réguler la population enzymatique de façon à contrôler la quantité de glucose consommée.

Les figures 3A et 3B représentent un exemple de réalisation d'un actionneur osmotique 50 selon l'invention mettant en oeuvre le premier exemple de réalisation du dispositif 10 de variation de pH. L'actionneur osmotique 50 comprend une enveloppe 52 déformable et étanche remplie d'un solvant et reliée au dispositif 10 au niveau de la vanne 16. L'enveloppe 52 est par ailleurs reliée par l'intermédiaire d'une membrane 54 à un corps cylindrique 56 dans lequel peut coulisser un piston mobile 58. Le piston mobile 58 et le corps cylindrique 56 définissent une chambre de dilatation 60. L'enveloppe 52 peut être disposée dans un boîtier rigide 64 qui est perforé pour que les déformations de l'enveloppe 52 ne soient pas gênées par un vide qui pourrait s'installer entre l'enveloppe 52 et le boîtier 64, si ce dernier était étanche.

La chambre de dilatation 60 contient une substance A, dissoute dans le solvant et osmotiquement active et à une concentration permettant un équilibre osmotique avec l'intérieur de l'enveloppe 52. La membrane 54 a un seuil de coupure suffisamment faible pour bloquer la substance A. A titre d'exemple, la substance A est le dextrane. Le dispositif 10 de variation de pH est du type permettant de rendre basique la solution qu'il contient, comme cela a été décrit précédemment. L'enveloppe 52 contient une substance Z osmotiquement active. La membrane 54 a un seuil de coupure suffisamment faible pour bloquer la substance Z. A titre d'exemple, la substance Z est le chitosane, soluble dans l'eau à pH acide, et insoluble à pH basique. La variation du pH de la solution entraîne donc la variation du nombre de molécules en solution et par conséquent de l'osmolarité de la solution.

Le fonctionnement de l'actionneur osmotique 50 selon l'invention va maintenant être décrit. Il s'agit d'un actionneur à un seul coup.

La figure 3A représente l'actionneur osmotique 50 dans l'état qui précède le fonctionnement de l'actionneur. La vanne 16 est alors fermée. L'enveloppe 52 a un volume maximum tandis que la chambre de dilatation 60 a un volume minimum. Les concentrations des substances A et Z sont initialement choisies de sorte que les pressions osmotiques dans la chambre de dilatation 60 et dans l'enveloppe 52 s'équilibrent, le piston 58 restant fixe. Avant le fonctionnement de l'actionneur 50, le dispositif 10 de variation de pH est commandé, comme cela a été décrit précédemment, de sorte que l'enceinte 12 contient une solution basique.

Lorsqu'on souhaite faire fonctionner l'actionneur osmotique 50, la vanne 16 est ouverte. Les ions OH⁻ contenus dans l'enceinte 12 se répandent dans l'enveloppe 52 entraînant une augmentation du pH du solvant contenu dans l'enveloppe 52. Lorsque le pH dans l'enveloppe 52 a augmenté jusqu'à la valeur souhaitée, la vanne 16 est refermée. La précipitation de la substance Z est alors favorisée. La pression osmotique dans l'enveloppe 52 diminue par rapport à la pression osmotique dans la chambre de dilatation 60 qui ne varie pas. Un transfert de solvant se produit donc, du solvant passant de l'enveloppe 52 à la chambre de dilatation 60 en traversant la membrane 54, entraînant le déplacement du piston 58. Le piston 58 est en phase ascendante.

La figure 3B représente l'actionneur osmotique 50 à la fin de la phase ascendante du piston 58. La chambre de dilatation 60 a alors un volume maximum. Le piston 58 peut être relié à un élément extérieur auquel on souhaite transmettre une énergie mécanique.

Dans l'exemple d'actionneur 50 décrit précédemment, le dispositif 10 est du type permettant de rendre basique la solution qu'il contient. Toutefois, l'actionneur 50 peut également être mis en oeuvre avec un dispositif 10 adapté à rendre acide la solution qu'il contient. Dans ce cas, initialement, la chambre de dilatation 60 peut avoir un volume maximum et l'enveloppe 52 un volume minimum comme cela est représenté en figure 3B. Lorsque la vanne 16 est ouverte, le pH du solvant dans l'enveloppe 52 et la chambre de dilatation 60 diminue, favorisant la solubilisation de la substance Z présente dans l'enveloppe 52. La pression osmotique dans l'enveloppe 52 augmente par rapport à la pression osmotique dans la chambre de dilatation 60 qui ne varie pas. Un transfert de solvant se produit donc, du solvant passant de la chambre de dilatation 60 dans l'enveloppe 52 en traversant la membrane 54, entraînant, par succion, le déplacement du piston 58. Le piston 58 est alors en phase descendante jusqu'à ce que la chambre de dilatation 60 ait un volume minimum comme cela est représenté en figure 3A.

Un exemple d'application de l'actionneur 50 consiste au gonflage d'un joint associé à une endoprothèse correspondant à un petit ressort, ou stent, glissé dans une cavité du corps humain (artère, par exemple) pour la maintenir ouverte. En effet, des joints peuvent être prévus aux extrémités du stent et être éventuellement gonflés, après la pose du stent, au moyen de l'actionneur 50 pour maintenir l'étanchéité aux extrémités du stent dans le cas d'une déformation ultérieure et indésirable de la cavité contenant le stent.

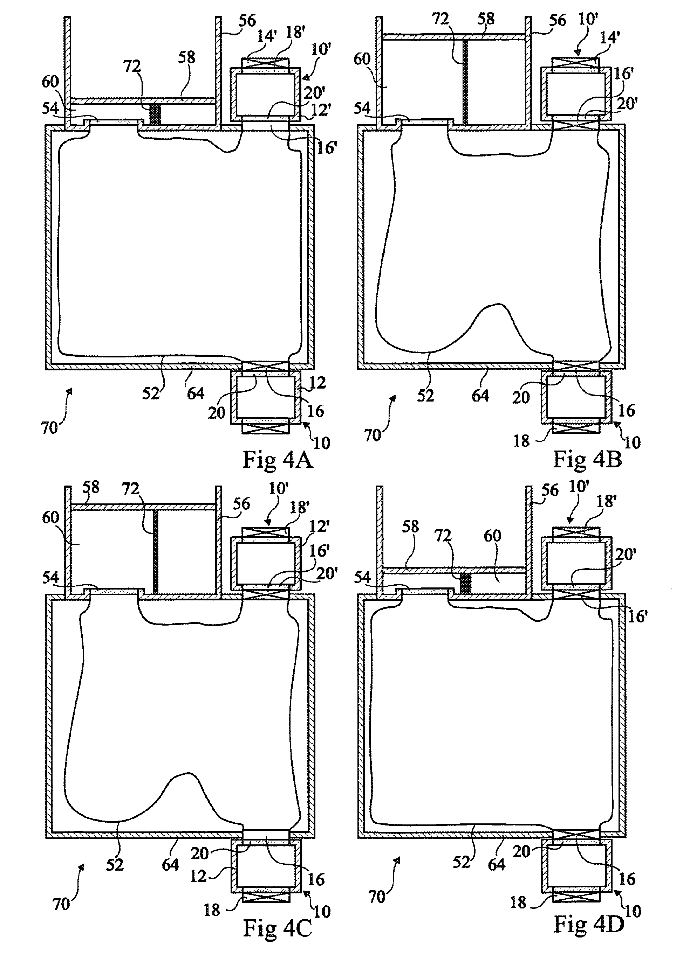

Les figures 4A à 4D représentent un premier exemple de réalisation d'un moteur osmotique 70 selon l'invention. Le moteur osmotique 70 inclut certains composants de l'actionneur 50 représenté aux figures 3A et 3B et les références correspondantes sont conservées. Un moyen de rappel 72, par exemple un ressort, exerce sur le piston 58 un effort de traction tendant à le ramener dans une position de repos. Le moteur 70 comprend deux dispositifs de variation de pH, notés 10 et 10'. Dans la suite de la description, on utilise des références primes pour désigner les éléments du dispositif de variation du pH 10', de façon à les distinguer des éléments du dispositif de variation de pH 10. L'enveloppe 52 est reliée au dispositif 10' au niveau de la vanne 16'. Le dispositif 10 est du type permettant la diminution du pH de la solution qu'il contient et le dispositif 10' est du type permettant l'augmentation du pH de la solution qu'il contient.

L'enveloppe 52 contient, tel que décrit précédemment, la substance Z susceptible de se solubiliser en présence d'ions $H^+$, la réaction inverse selon laquelle la substance Z précipite étant susceptible de se produire en présence d'ions $OH^-$. Comme cela a été décrit précédemment pour l'actionneur osmotique 50, la chambre de dilatation 60 contient une substance A, dissoute dans le solvant, osmotiquement active. La membrane 54 a un seuil de coupure suffisamment faible pour empêcher le passage des substances A et Z.

Un cycle de fonctionnement du moteur 70 selon l'invention va maintenant être décrit.

La figure 4A représente le moteur 70 au début d'un cycle. L'enveloppe 52 a un volume maximum et la chambre 60 a un volume minimum. Les concentrations des substances A et Z sont initialement choisies de sorte que les pressions osmotiques dans la chambre de dilatation 60 et dans l'enveloppe 52 s'équilibrent, le piston 58 restant fixe. Parallèlement au cycle d'ouvertures des vannes 16 et 16' qui va maintenant être décrit, les dispositifs 10, 10' sont commandés pour que les enceintes 12, 12' contiennent des solutions ayant le pH désiré.

La vanne 16 est fermée et la vanne 16' est ouverte. Les ions $OH^-$ se répandent dans l'enveloppe 52 entraînant une augmentation du pH du solvant contenu dans l'enveloppe 52 et dans la chambre de dilatation 60. Lorsque le pH dans l'enveloppe 52 a augmenté jusqu'à la valeur souhaitée, la vanne 16' est fermée. La précipitation de la substance Z est alors favorisée. La pression osmotique dans l'enveloppe 52 diminue par rapport à la pression osmotique dans la chambre de dilatation 60 qui ne varie pas. Un transfert de solvant se produit donc, du solvant passant de l'enveloppe 52 à la chambre de dilatation 60 en traversant la membrane 54, entraînant le déplacement du piston 58. Le piston 58 est en phase ascendante.

La figure 4B représente le moteur 70 à la fin de la phase ascendante du piston 58. La chambre 60 a alors un volume maximum.

En figure 4C, on a ouvert la vanne 16 de façon à mettre en communication le contenu de l'enveloppe 52 avec le contenu de l'enceinte 12. Des ions $H^+$ se répandent dans l'enveloppe 52 entraînant une diminution du pH du solvant contenu dans l'enveloppe 52 ainsi que dans la chambre de dilatation 60. Lorsque le pH est suffisamment acide, la vanne 16 est fermée. La solubilisation de la substance Z est alors favorisée, entraînant l'augmentation du nombre de particules osmotiquement actives en solution dans l'enveloppe 52. La pression osmotique augmentant dans l'enveloppe 52, un nouveau transfert de solvant se produit, du solvant passant de la chambre de dilatation 60 vers l'enveloppe 52 par l'intermédiaire de la membrane 54. L'action du ressort 72 favorise l'évacuation du solvant de la chambre de dilatation 60. Toutefois, le ressort 72 pourrait ne pas être présent, le piston 58 se déplaçant seulement par succion. Le piston 58 est dit en phase descendante.

La figure 4D représente le moteur 70 à la fin de la phase descendante du piston 58, ce qui clôt le cycle. Les concentrations des substances Z et A respectivement dans l'enveloppe 52 et la chambre de dilatation 60 sont alors sensiblement identiques aux concentrations en début de cycle.

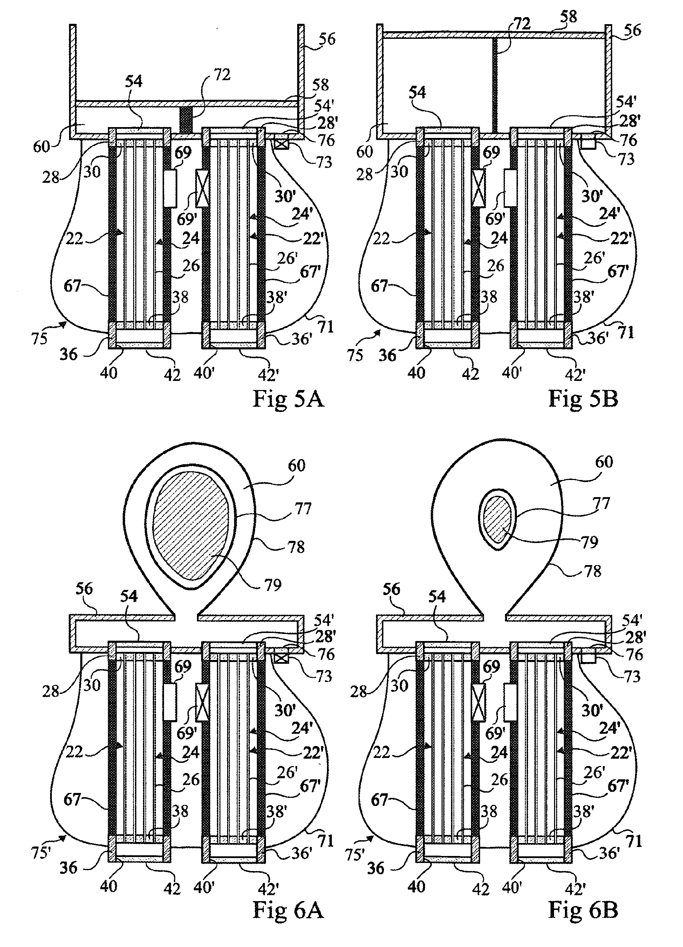

Les figures 5A et 5B représentent un second exemple de réalisation d'un moteur osmotique 75 mettant en oeuvre le second exemple de réalisation du dispositif 22 de variation de pH et certains éléments du moteur osmotique 70. Le moteur 75 comprend deux dispositifs de variation de pH, notés 22 et 22'. Dans la suite de la description, on utilise des références primes pour désigner les éléments du dispositif de variation du pH 22', de façon à les distinguer des éléments du dispositif de variation de pH 22. Chaque dispositif de variation de pH 22, 22' communique avec la chambre de dilatation 60 au niveau de la membrane 54, 54' qui remplace le bouchon 34. En outre, le dispositif de variation de pH 22 est contenu dans une enceinte 67 étanche qui relie les bagues de jonction 28 et 36 et le dispositif de variation de pH 22' est contenu dans une enceinte 67' étanche qui relie les bagues de jonction 28' et 36'. Une vanne 69, 69' est prévue au niveau de l'enceinte 67, 67'. Les enceintes 67, 67' sont contenues dans une enveloppe 71 semi-perméable qui est reliée au corps cylindrique 56 et aux bagues de jonction 36, 36' des dispositifs de variation de pH 22, 22'. Le corps cylindrique 56 comprend une vanne d'évacuation 73 qui relie la chambre de dilatation avec l'extérieur du moteur 75 par l'intermédiaire d'une membrane 76.

A titre d'exemple, le dispositif 22' est du type permettant la diminution du pH de la solution dans laquelle il est disposé selon les réactions (1) décrites précédemment, des enzymes glucose oxydase étant disposées dans les fibres 26'. Le dispositif 22 est du type permettant l'augmentation du pH de la solution dans laquelle il est disposé selon les réactions (3) décrites précédemment, des enzymes uréase étant disposées dans les fibres 26.

On dispose en outre, dans l'enveloppe 71, le composé Z, tel que précédemment décrit, susceptible de se solubiliser en milieu acide. Le seuil de coupure des fibres 26 permet d'empêcher le passage des substances A et Z, et des enzymes uréase. Le seuil de coupure de la membrane 54 permet d'empêcher le passage des enzymes uréase mais permet le passage de la substance A. En outre, le seuil de coupure des fibres 26' permet d'empêcher le passage des substances A et Z, et des enzymes glucose oxydase. Le seuil de coupure de la membrane 54' permet d'empêcher le passage des enzymes glucose oxydase mais permet le passage de la substance A. Le seuil de coupure de l'enveloppe 71 permet d'empêcher le passage de la substance Z. Le seuil de coupure de la membrane 76 permet d'empêcher le passage de la substance A.

En fonctionnement normal, le moteur 75 est placé dans une solution contenant du glucose et de l'urée. Le seuil de coupure des fibres 26' est suffisamment élevé pour permettre le passage du glucose et le seuil de coupure des fibres 26 est suffisamment élevé pour permettre le passage de l'urée. En outre, le seuil de coupure de l'enveloppe 71 est suffisamment élevé pour permettre le passage du solvant, du glucose et de l'urée. Les concentrations des substances A et Z sont initialement choisies de sorte que les pressions osmotiques dans la chambre de dilatation 60 et dans l'enveloppe 71 s'équilibrent, le piston 58 restant fixe.

Un cycle de fonctionnement du moteur 75 va maintenant être décrit.

La figure 5A représente le moteur 75 au début d'un cycle. Le piston 58 est en position de repos, le volume de la chambre de dilatation 60 étant minimal. La vanne d'évacuation 73 est fermée, la vanne 69' est fermée et la vanne 69 est ouverte. Dès que le moteur 75 est placé dans la solution contenant de l'urée, l'urée traverse l'enveloppe 71 et se répand jusque dans les fibres 26. La dégradation de l'urée conduit à l'obtention d'ions OH- qui se répandent dans l'enveloppe 71. L'augmentation du pH dans l'enveloppe 71 favorise la précipitation de la substance Z ce qui tend à faire baisser la pression osmotique dans l'enveloppe 71. La variation de pression osmotique entraîne un flux de liquide de l'intérieur de l'enveloppe 71 vers la chambre de dilation 60 par l'intermédiaire des fibres 26, déplaçant ainsi le piston 58. Le déplacement du piston 58 tend le ressort 72, permettant d'emmagasiner de l'énergie mécanique.

Sur la figure 5B, la chambre de dilatation 60 est représentée en expansion maximale. La vanne 69 est alors fermée et la vanne 69' est ouverte. En outre, la vanne d'évacuation 73 est ouverte. La pression à l'intérieur de la chambre de dilatation 60 s'égalise avec la pression du milieu ambiant. Le ressort 72 ramène le piston 58 en position de repos en évacuant, par la vanne d'évacuation 73, le solvant de la chambre de dilatation 60 dans le milieu ambiant. L'énergie mécanique emmagasinée dans le ressort 72 est ainsi récupérée. Par ailleurs, la vanne 69' étant ouverte, du glucose pénètre dans les fibres 26'. La dégradation du glucose conduit à l'obtention d'ions H+ qui se répandent dans l'enveloppe 71. La baisse du pH dans l'enveloppe 71 favorise la solubilisation de la substance Z, ce qui permet de retrouver les concentrations de la substance Z du début du cycle. La vanne 73 est finalement fermée, achevant ainsi le cycle moteur.

Dans le présent exemple de réalisation, la chambre de dilatation 60 est définie par un corps cylindrique 56 dans lequel coulisse un piston 58. En fonction de l'utilisation souhaitée du moteur 75 selon l'invention, la chambre de dilatation 60 peut être réalisée de façon différente.

Les figures 6A et 6B représentent un moteur osmotique 75' mettant en oeuvre une variante de structure de la chambre de dilatation 60 du moteur 75 de l'exemple de réalisation précédent. Selon cette variante, la chambre de dilatation 60 correspond à l'espace défini entre une enveloppe intérieure 77 et une enveloppe extérieure 78 à la manière d'une chambre à air. L'enveloppe intérieure 77 est déformable et extensible et entoure un corps déformable 79. L'enveloppe extérieure 78 est souple et inextensible. Elle se referme sur l'enveloppe intérieure 77 et est reliée au corps cylindrique 56. A titre d'exemple, dans une application médicale du moteur osmotique 75' selon l'invention, le corps déformable 79 peut être un organe creux du corps humain, tel que l'estomac, l'urètre, l'anus ou le coeur et les enveloppes 77, 78 peuvent définir des chambres de dilatation 60 en forme de boudins entourant l'organe creux. Le moteur osmotique joue alors le rôle d'une source motrice d'un sphincter artificiel (lorsque l'organe creux est l'urètre ou l'anus) ou d'un anneau gastrique commandable (lorsque l'organe creux est l'estomac).

Un cycle du moteur 75' selon la présente variante va maintenant être décrit.

La figure 6A représente le moteur 75' en début de cycle. Le volume de la chambre de dilatation 60 est minimal, le corps déformable 79 étant en dilation maximale, ce qui peut correspondre à un urètre ou à un canal anal ou à un estomac "ouverts" au maximum ou à un coeur en diastole. La vanne d'évacuation 73 est fermée, la vanne 69' est fermée et la vanne 69 est ouverte. Les enzymes uréase favorisent la dégradation de l'urée et l'augmentation du pH dans l'enveloppe 71, ce qui favorise la précipitation de la substance Z. Ceci entraîne, par osmose l'introduction de solvant dans la chambre de dilatation 60. L'enveloppe intérieure 77 se déforme et comprime le corps déformable 79.

Sur la figure 6B, le corps déformable 79 est comprimé au maximum, ce qui peut correspondre à un urètre "fermé", à un canal anal "fermé", à un estomac "ouvert" au minimum ou à un coeur en systole. La vanne 69' est alors ouverte et la vanne 69 est fermée. A l'ouverture de la vanne d'évacuation 73, le solvant évacue la chambre de dilatation 60 permettant la dilation du corps déformable 79, ce qui achève le cycle.

Selon une autre variante de l'invention, la chambre de dilatation est constituée d'une enveloppe supplémentaire élastique enfermant des fibres qui sont disposées, par exemple, en spirale et qui sont reliées à une extrémité au corps cylindrique 56. Lorsque du solvant pénètre dans les fibres, celles-ci ont tendance à se redresser et à déformer l'enveloppe élastique. A l'ouverture de la vanne 73, la pression à l'intérieur des fibres diminue et l'enveloppe supplémentaire tend à reprendre sa forme initiale.

Selon une autre variante de l'invention, le dispositif de variation de pH peut être relié à la chambre déformable par un conduit souple. Ceci permet de disposer de façon avantageuse le dispositif de variation de pH dans un milieu ambiant propice pour l'apport en solvant dans lequel sont dissoutes les substances utilisées (glucose ou urée par exemple) par les enzymes contenues dans le dispositif de variation de pH, et de placer la chambre de dilatation à un endroit où l'on souhaite disposer de l'énergie mécanique. Dans le cas d'une application médicale, on pourra disposer le dispositif de variation de pH dans un tissu graisseux, ou sur le réseau vasculaire. Dans ce dernier cas, les fibres peuvent être agencées pour former un tube creux, laissant en son centre un espace cylindrique permettant la circulation d'un fluide tel que le sang. Les bagues de jonction peuvent être de forme torique et placées contre la paroi d'un vaisseau sanguin. L'une des bagues de jonction toriques communique avec la chambre de dilatation par le conduit souple qui perfore le vaisseau sanguin.

Les figures 7A et 7B représentent une variante du dispositif 10 de variation de pH selon le premier exemple de réalisation de l'invention. Selon une telle variante, on prévoit dans l'enceinte 12 un piston mobile 81 adapté à coulisser dans l'enceinte 12. Le piston mobile 81 est entraîné par un moteur (M) 82, par exemple le moteur osmotique 70 décrit en relation aux figures 4A à 4D. La référence 84 désigne un élément de liaison entre le piston mobile 81 et le moteur 82, par exemple un moyen de rappel. Le piston mobile 81 et l'enceinte 12 définissent une chambre de volume variable 86. Une conduite d'évacuation 88 est reliée à la vanne 16.

La réaction privilégiée dans la chambre 86 est la réaction d'augmentation du pH par dégradation de l'urée (réactions (3)). Des enzymes uréase sont alors disposées dans la chambre 86. Les membranes 18, 20 ont un seuil de coupure permettant de retenir les enzymes uréase dans la chambre 86. Le dispositif 80 est adapté à une application selon laquelle, parallèlement à l'augmentation du pH, on souhaite éliminer de l'urée en excès dans la solution dans laquelle le dispositif 80 est placé.

Un cycle de fonctionnement du dispositif 80 selon l'invention va maintenant être décrit.

La figure 7A représente le dispositif 80 au début d'un cycle. Le volume de la chambre 86 est maximum, la vanne 14 est ouverte et la vanne 16 est fermée. La solution contenue dans la chambre 86 correspond sensiblement à la solution dans laquelle le dispositif 80 est disposé. La vanne 14 est alors fermée. La réaction de dégradation de l'urée se produit dans la chambre 86 entraînant une augmentation du pH de la solution contenue dans la chambre 86. Le moteur 82 est alors actionné pour déplacer le piston 81 entraînant une diminution du volume de la chambre 86. La vanne 16 est ouverte lors du déplacement du piston 81 entraînant l'expulsion de la solution contenue dans la chambre 86 par l'intermédiaire de la conduite 88.

La figure 7B représente le dispositif 80 à la fin de la phase ascendante du piston 81. La chambre 86 a alors un volume minimum. La vanne 16 est alors fermée. Le moteur 82 est alors actionné pour déplacer le piston 81 entraînant une augmentation du volume de la chambre 86. La vanne 14 est ouverte lors du déplacement du piston 81 entraînant la succion dans la chambre 86 de la solution dans laquelle est placé le dispositif 80. Le cycle s'achève lorsque la chambre 86 à un volume maximum.

Un tel dispositif 80 peut être implanté dans le corps humain pour l'élimination d'un excès d'urée. Les ions ammonium obtenus lors de la dégradation de l'urée sont alors susceptibles de réagir avec des éléments tels que le magnésium, le calcium et le potassium présents dans une solution biologique pour former des composés solides. Il peut alors ne pas être souhaitable de rejeter de tels composés solides hors du dispositif 80 dans la solution biologique. Pour ce faire, la conduite 88 est reliée à une région susceptible de recevoir les déchets issus de la dégradation de l'urée, par exemple au niveau du colon.

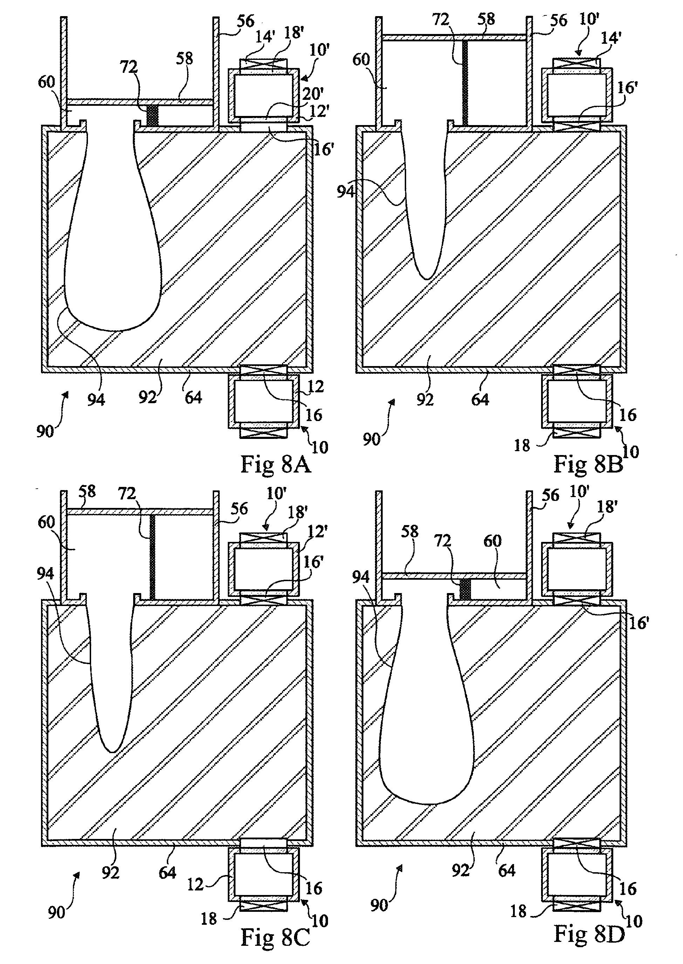

Les figures 8A à 8D représentent un troisième exemple de réalisation d'un moteur osmotique 90 selon l'invention. Le moteur osmotique 90 inclut certains composants du premier exemple de moteur osmotique 70 représenté aux figures 4A à 4D et les références correspondantes sont conservées.

Par rapport au moteur osmotique 70, l'enveloppe 52 et la membrane 54 ne sont pas présentes. Le boîtier 64 contient un gel polymère 92 capable de changer de volume en fonction du pH. Il s'agit par exemple de gel polymère réversible en fonction du pH tel que décrit dans les travaux de Y. Osada (Polymer Gels and Networks, Yoshihito Osada and Alexi R. Khokhlov, New York, Marcel Dekker Inc., 2001, 400 p.) qui augmente de volume lorsque le pH augmente. Une enveloppe étanche 94 est disposée dans le boîtier 92 et communique avec la chambre de dilatation 60. L'enveloppe 94 et la chambre de dilatation 60 sont remplies d'un solvant, par exemple une solution aqueuse.

Un cycle de fonctionnement du moteur 90 va maintenant être décrit.

La figure 8A représente le moteur 90 au début d'un cycle. Le gel 92 occupe un volume minimum. L'enveloppe 94 a donc un volume maximum et la chambre 60 a un volume minimum. Parallèlement au cycle d'ouvertures des vannes 16 et 16' qui va maintenant être décrit, les dispositifs 10, 10' sont commandés pour que les enceintes 12, 12' contiennent des solutions ayant le pH désiré.

La vanne 16 est fermée et la vanne 16' est ouverte. Les ions OH- se répandent dans le boîtier 64 entraînant une augmentation du pH. Le gel 92 augmente alors de volume et comprime l'enveloppe 94. Lorsque le pH dans l'enveloppe 52 a augmenté jusqu'à la valeur souhaitée, la vanne 16' est fermée. La diminution du volume de l'enveloppe 94 entraîne un transfert de solvant de l'enveloppe 94 vers la chambre de dilatation 60, entraînant le déplacement du piston 58. Le piston 58 est en phase ascendante.

La figure 8B représente le moteur 90 à la fin de la phase ascendante du piston 58. La chambre 60 a alors un volume maximum.

En figure 8C, on a ouvert la vanne 16 de façon à mettre en communication le contenu du boîtier 64 avec le contenu de l'enceinte 12. Des ions $H^+$ se répandent dans le boîtier 64 entraînant une diminution du pH dans le boîtier 64. Lorsque le pH est suffisamment acide, la vanne 16 est fermée. La diminution du volume du gel 92 est alors favorisée, entraînant l'augmentation du volume de l'enveloppe 94. Un nouveau transfert de solvant se produit, du solvant passant de la chambre de dilatation 60 vers l'enveloppe 94. L'action du ressort 72 favorise l'évacuation du solvant de la chambre de dilatation 60. Toutefois, le ressort 72 pourrait ne pas être présent, le piston 58 se déplaçant seulement par succion. Le piston 58 est dit en phase descendante.

La figure 8D représente le moteur 90 à la fin de la phase descendante du piston 58, ce qui clôt le cycle.

L'utilisation du gel 92 dont le volume varie en fonction du pH peut être mise en oeuvre pour la réalisation d'un actionneur à coup unique. A titre d'exemple, par rapport au moteur 90, seul le dispositif 10' est présent. Le fonctionnement de l'actionneur est alors identique à ce qui a été décrit précédemment pour le moteur 90 en relation aux figures 8A et 8B. Selon un autre exemple, par rapport au moteur 90, seul le dispositif 10 est présent. Le fonctionnement de l'actionneur est alors identique à ce qui a été décrit précédemment pour le moteur 90 en relation aux figures 8C et 8D.

Selon une variante des actionneurs et moteurs osmotiques 70, 90 décrits précédemment, les membranes 20, 20' et 54 peuvent être remplacées par un faisceau de fibres ou un système analogue s'il est souhaitable d'augmenter la surface d'échange entre le contenu de l'enceinte 12, 12' et le contenu du boîtier 64 ou entre le contenu de l'enveloppe 52 et le contenu de la chambre de dilatation 60.

Bien entendu, la présente invention est susceptible de diverses variantes et modifications qui apparaîtront à l'homme de l'art. En particulier, la variante de réalisation de la chambre de dilatation du moteur osmotique décrit en relation aux figures 6A et 6B peut être appliquée au moteur osmotique décrit en relation aux figures 4A à 4D.

**Revendications**

1.  Actionneur (50) comprenant :

    une première chambre (52 ; 24 ; 64) contenant au moins partiellement un solvant ; et
    une seconde chambre déformable (60) reliée à la première chambre,
    **caractérisé en ce que** l'actionneur comprend, en outre :

    un dispositif (10 ; 22) de variation de pH du solvant dans lequel est dissous une substance, comprenant une enceinte (12 ; 24) en contact avec le solvant par l'intermédiaire d'une paroi d'enceinte (18 ; 26) perméable au solvant et à la substance, l'enceinte contenant des enzymes adaptées à transformer la substance pour fournir des ions hydrogène ou hydroxyle, la paroi d'enceinte étant non perméable aux enzymes, la première chambre (52 ; 24 ; 64) étant reliée au dispositif de variation de pH ou correspondant à l'enceinte (24) du dispositif de variation de pH, le dispositif de variation de pH étant adapté à amener le pH dans la première chambre dans une plage de pH donnée, la première chambre contenant un moyen (52 ; 92) adapté à transférer du solvant entre la première chambre et la seconde chambre lorsque le pH dans la première chambre est dans la plage de pH donnée.

2.  Actionneur (50) selon la revendication 1, dans lequel la première chambre contient un soluté et est séparée de la seconde chambre (60) par une paroi de chambre (54) non perméable au soluté et perméable au solvant, la transformation d'un précipité dudit soluté en ledit soluté ou la précipitation du soluté étant favorisée lorsque le pH dans la première chambre est dans la plage de pH donnée pour faire varier la pression osmotique dans la première chambre et dans lequel la seconde chambre déformable est adaptée à changer de volume sous l'action du solvant se déplaçant entre la première chambre et la seconde chambre par osmose.

**3.** Actionneur selon la revendication 1, dans lequel la première chambre contient un soluté et est séparée de la seconde chambre (60) par une paroi de chambre (26) non perméable au soluté et perméable au solvant, la transformation d'un précipité dudit soluté en ledit soluté étant favorisée lorsque le pH dans la première chambre est dans la plage de pH donnée pour faire varier la pression osmotique dans la première chambre et dans lequel la première chambre est destinée à être disposée au contact du solvant, la seconde chambre étant adaptée à augmenter de volume sous l'action du solvant pénétrant dans la première chambre par osmose.

**4.** Actionneur selon la revendication 1, dans lequel la première chambre (64) comprend un matériau (92) adapté à changer de volume lorsque le pH dans la première chambre est dans la plage donnée, le matériau entourant une enveloppe (94) déformable contenant du solvant et reliée à la seconde chambre (60).

**5.** Moteur (70) comprenant un actionneur selon la revendication 1, dans lequel la première chambre (52) est au moins en partie déformable et est reliée à la seconde chambre (60) au niveau de la paroi de chambre (54), la transformation du précipité dudit soluté en ledit soluté étant favorisée pour un pH compris dans une plage de pH acide donnée, et la précipitation dudit soluté étant favorisée pour un pH compris dans une plage de pH basique donnée, le dispositif de variation de pH (10) étant adapté à amener le pH dans la première chambre dans la plage de pH acide donné, le moteur comprenant, en outre, un dispositif de variation de pH (10') supplémentaire relié à la première chambre et adapté à amener le pH dans la première chambre dans la plage de pH basique donné.

**6.** Moteur (75 ; 75') comprenant un actionneur selon la revendication 3, dans lequel la seconde chambre (60) comporte un moyen de rappel (72) qui s'oppose à l'augmentation du volume de la seconde chambre et un moyen commandable (76) pour faire baisser la pression osmotique dans la seconde chambre.

**7.** Moteur (90) comprenant un actionneur selon la revendication 4, dans lequel le matériau (92) est adapté à diminuer de volume pour un pH compris dans une plage de pH acide donnée, et à augmenter de volume pour un pH compris dans une plage de pH basique donnée, le dispositif de variation de pH (10) étant adapté à amener le pH dans la première chambre dans la plage de pH acide donné, le moteur comprenant, en outre, un dispositif de variation de pH (10') supplémentaire relié à la première chambre et adapté à amener le pH dans la première chambre dans la plage de pH basique donné.

**8.** Moteur (75') selon l'un quelconque des revendications 5 à 7, dans lequel la seconde chambre (60) est destinée à entourer un organe creux (79) d'un mammifère, notamment l'urètre, l'estomac, l'anus ou le coeur, une augmentation du volume de la seconde chambre entraînant une constriction dudit organe.

**Claims**

**1.** An actuator (50) comprising:

a first chamber (52; 24; 64) at least partially containing a solvent; and
a second deformable chamber (60) connected to the first chamber,
**characterized in that** the actuator further comprises:

a device (10; 22) for varying the pH of a solvent in which a substance is dissolved, comprising an enclosure (12; 24) in contact with the solvent via an enclosure wall (18; 26) permeable to the solvent and to the substance, the enclosure containing enzymes capable of transforming the substance to release hydrogen or hydroxyl ions, the enclosure wall being non-permeable to enzymes, the first chamber (52; 24; 64) being connected to the device for varying the pH or corresponding to the enclosure (24) of the device for varying the pH, the device for varying the pH being capable of bringing the pH in the first chamber within a given pH range, the first chamber containing means (52; 92) capable of transferring solvent between the first chamber and the second chamber when the pH in the first chamber is within the given pH range.

2. The actuator (50) of claim 1, wherein the first chamber contains a solute and is separated from the second chamber (60) by a chamber wall (54) non-permeable to the solute and permeable to the solvent, the transformation of a precipitate of said solute into said solute or the solute precipitation being promoted when the pH in the first chamber is within the given pH range to vary the osmotic pressure in the first chamber, and wherein the second deformable chamber is capable of changing volume under the action of the solvent displacing between the first chamber and the second chamber by osmosis.

3. The actuator of claim 1, wherein the first chamber contains a solute and is separated from the second chamber (60) by a chamber wall (26) non-permeable to the solute and permeable to the solvent, the transformation of a precipitate of said solute into said solute being promoted when the pH in the first chamber is within the given pH range to vary the osmotic pressure in the first chamber and wherein the first chamber is intended to be arranged in contact with the solvent, the second chamber being capable of increasing its volume under the action of the solvent penetrating into the first chamber by osmosis.

4. The actuator of claim 1, wherein the first chamber (64) comprises a material (92) capable of changing volume when the pH in the first chamber is within the given range, the material surrounding a deformable envelope (94) containing solvent and coupled with the second chamber (60).

5. A motor (70) comprising the actuator of claim 1, wherein the first chamber (52) is at least partly deformable and is connected to the second chamber (60) at the level of the chamber wall (54), the transformation of the precipitate of said solute into said solute being promoted for a pH within a given acid pH range, and the precipitation of said solute being promoted for a pH within a given basic pH range, the device for varying the pH (10) being capable of bringing the pH in the first chamber within the given acid pH range, the motor further comprising an additional device for varying the pH (10') connected to the first chamber and capable of bringing the pH in the first chamber within the given basic pH range.

6. A motor (75; 75') comprising the actuator of claim 3, wherein the second chamber (60) comprises return means (72) which oppose the volume increase of the second chamber and controllable means (76) for lowering the osmotic pressure in the second chamber.

7. A motor (90) comprising the actuator of claim 4, wherein the material (92) is capable of decreasing its volume for a pH ranging within a given acid pH range, and of increasing its volume for a pH within a given basic pH range, the device for varying the pH (10) being capable of bringing the pH in the first chamber within the given acid pH range, the motor further comprising an additional device for varying the pH (10') connected to the first chamber and capable of bringing the pH in the first chamber within the given basic pH range.

8. The motor (75') of any of claims 5 to 7, wherein the second chamber (60) is intended to surround a hollow organ (79) of a mammal, especially the urethra, the stomach, the anus, or the heart, a volume increase of the second chamber causing a constriction of said organ.

**Patentansprüche**

1. Ein Aktuator (Aktor) (50) der Folgendes aufweist:

    eine erste Kammer (52; 24; 64), die mindestens teilweise ein Lösungsmittel enthält; und
    eine zweite deformierbare Kammer (60), die mit der ersten Kammer verbunden ist, **dadurch gekennzeichnet, dass** der Aktuator ferner Folgendes aufweist:

    eine Vorrichtung (10; 22) zum Verändern des pH-Werts eines Lösungsmittels in dem eine Substanz aufgelöst ist, eine Umschließung (12; 24) aufweisend und zwar in Kontakt mit dem Lösungsmittel über eine Umschließungswand (18; 26) permeabel für das Lösungsmittel und die Substanz, wobei die Umschließung Enzyme enthält, die in der Lage sind die Substanz umzuwandeln um Wasserstoff- oder Hydroxyl-Ionen freizugeben, wobei die Umschließungswand für Enzyme nicht permeabel ist,
    wobei
    die erste Kammer (52; 24; 64) mit der Vorrichtung zur Veränderung des pH-Wertes oder entsprechend mit der Umhüllung (24) der Vorrichtung verbunden ist, um den pH-Wert zu verändern, wobei die Vorrichtung zur Veränderung des pH-Wertes in der Lage ist, den pH-Wert in der ersten Kammer in einen vorgegebenen pH-Bereich zu bringen, wobei die erste Kammer Mittel (52; 92) enthält, die in der Lage sind, das Lösungsmittel zwischen der ersten Kammer und der zweiten Kammer zu transferieren, wenn der pH-Wert in der ersten Kammer innerhalb des gegebenen pH-Bereichs liegt.

2.  Aktuator (50) nach Anspruch 1, wobei die erste Kammer einen gelösten Stoff enthält und von der zweiten Kammer (60) durch eine Kammerwand (54) getrennt ist, die für den gelösten Stoff nicht permeabel und für das Lösungsmittel permeabel ist, wobei die Transformation einer Ausscheidung des gelösten Stoffs in den gelösten Stoff oder die Ausscheidung des gelösten Stoffs gefördert wird, wenn der pH-Wert in der ersten Kammer innerhalb des gegebenen pH-Bereichs liegt, um den osmotischen Druck in der ersten Kamme zu variieren, und wobei die zweite deformierbare Kammer in der Lage ist, das Volumen zu ändern und zwar unter der Wirkung des zwischen der ersten Kammer und der zweiten Kammer durch Osmose versetzten Lösungsmittels.

3.  Aktuator nach Anspruch 1, wobei die erste Kammer einen gelösten Stoff enthält und getrennt ist von der zweiten Kammer (60) durch eine Kammerwand (26), die nicht permeabel ist für den gelösten Stoff und permeabel ist für das Lösungsmittel, wobei die Transformation einer Ausscheidung des gelösten Stoffs in den gelösten Stoff dann gefördert wird, wenn der pH-Wert in der ersten Kammer innerhalb eines gegebenen pH-Bereichs liegt, um den osmotischen Druck in der ersten Kammer zu variieren, und wobei die erste Kammer vorgesehen ist zur Anordnung in Kontakt mit dem Lösungsmittel, wobei die zweite Kammer in der Lage ist ihr Volumen zu vergrößern, und zwar unter der Wirkung des in die erste Kammer durch Osmose eindringenden Lösungsmittels.

4.  Aktuator nach Anspruch 1, wobei die erste Kammer (64) ein Material (92) aufweist, welches in der Lage ist das Volumen dann zu ändern, wenn der pH-Wert in der ersten Kammer innerhalb eines gegebenen Bereichs liegt, wobei das eine deformierbare Umschließung (94) umgebende Material Lösungsmittel enthält und mit der zweiten Kammer (60) gekoppelt ist.

5.  Ein Motor (70), der den Aktuator nach Anspruch 1 aufweist, wobei die erste Kammer (52) mindestens teilweise deformierbar ist und verbunden ist mit der zweiten Kammer (60) auf der Niveau der Kammerwand (54), wobei die Transformation der Ausscheidung des gelösten Stoffs in den gelösten Stoff gefördert wird für einen pH-Wert innerhalb eines gegebenen sauren pH-Bereichs, wobei die Ausscheidung des gelösten Stoffs gefördert wird für einen pH-Wert innerhalb eines gegebenen basischen pH-Bereichs, wobei die Vorrichtung zur Veränderung des pH-Werts (10) in der Lage ist, den pH-Wert in der ersten Kammer in den gegebenen sauren pH-Bereich zu bringen, und wobei der Motor ferner eine zusätzliche Vorrichtung aufweist zur Veränderung des pH-Werts (10') und zwar verbunden mit der ersten Kammer und in der Lage, den pH-Wert in der ersten Kammer in den gegebenen basischen pH-Bereich zu bringen.

6.  Ein Motor (75; 75'), der den Aktuator nach Anspruch 3 aufweist, wobei die zweite Kammer (60) Rückführmittel (72) aufweist, die dem Volumenanstieg der zweiten Kammer entgegenwirken und ferner steuerbare Mittel (76) zum Absenken des osmotischen Drucks in der zweiten Kammer.

7.  Ein Motor (90), der den Aktuator nach Anspruch 4 aufweist, wobei das Material (92) in der Lage ist, sein Volumen zu verringern und zwar für einen pH-Bereich innerhalb eines gegebenen sauren pH-Bereichs, und wobei das Material ferner in der Lage ist, sein Volumen zu vergrößern und zwar für einen pH-Wert innerhalb eines gegebenen basischen pH-Bereichs, wobei die Vorrichtung zum Verändern des pH-Werts (10) in der Lage ist, den pH-Wert in der ersten Kammer in einen gegebenen sauren pH-Bereich zu bringen, wobei der Motor ferner eine zusätzliche Vorrichtung aufweist zum Verändern des pH-Werts (10') und zwar verbunden mit der ersten Kammer und in der Lage ist, den pH-Wert in der ersten Kammer in den gegebenen basischen pH-Bereich zu bringen.

8.  Der Motor (75') nach einem der Ansprüche 5 bis 7, wobei die zweite Kammer (60) dazu vorgesehen ist ein hohles Organ (79) eines Säugetiers zu umgeben, und zwar insbesondere den Harnleiter, den Magen, den Anus oder das Herz, wobei ein Volumenanstieg der zweiten Kammer eine Einschränkung des erwähnten Organs bewirkt.

Fig 1

Fig 2

Fig 3A

Fig 3B

Fig 7A

Fig 7B

Fig 4A

Fig 4B

Fig 4C

Fig 4D

Fig 5A

Fig 5B

Fig 6A

Fig 6B

Fig 8A

Fig 8B

Fig 8C

Fig 8D

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 03072941 A
- FR 2881481
- DE 19728663

**Littérature non-brevet citée dans la description**

- **YOSHIHITO OSADA ; ALEXI R. KHOKHLOV.** Polymer Gels and Networks. Marcel Dekker Inc, 2001, 400